# EUROPÄISCHE PATENTANMELDUNG

(11) **EP 1 916 224 A1**
(43) Veröffentlichungstag der Anmeldung: **30.04.2008**
(21) Anmeldenummer: 07024692.1
(22) Anmeldetag: 31.10.2001
(51) Int. Cl.: C02F 1/32, B01J 19/12, A61L 2/10

(54) **Vorrichtung zur Bestrahlung von Flüssigkeiten**

(30) Priorität: 13.11.2000 DE 10056096
(62) Teilanmeldung aus: 01993380.3
(71) Anmelder: Bayer Technology Services GmbH, 51368 Leverkusen (DE)
(72) Erfinder: Kaiser, Klaus, Dr., 51105 Köln (DE); Kauling, Jörg, 51069 Köln (DE); Henzler, Hans-Jürgen, Dr., 42655 Solingen (DE); Günther, Isabell, 51381 Leverkusen (DE); Schmitt, Franz, 51465 Bergisch Gladbach (DE); Beckers, Erhard, 51399 Burscheid (DE); Quest, Stefan, Dr., 42799 Leverkusen (DE)

(57) **Zusammenfassung**

Es wird ein Reaktor zur Einstrahlung von ultraviolettem Licht in ein fluides Reaktionsmedium (3) beschrieben. Der Reaktor besteht wenigstens aus einem Gehäuse (15), welches einen rohrförmigen Hohlraum umschließt, mit einer Strahlungsquelle (1) zur Erzeugung von ultraviolettem Licht und einem inneren Rohr (2), das mit dem Gehäuse (15) einen insbesondere ringförmigen Bestrahlungsraum (26) bildet, wobei der Bestrahlungsraum (26) wenigstens mit einem Einlass (13) und einem Auslass (14) für das Reaktionsmedium (3) verbunden ist und vom Reaktionsmedium (3) in Längsrichtung des Rohres (2) durchströmt wird, und wobei der Bestrahlungsraum (26) Mittel (6, 25) zur Erzeugung einer zusätzlichen radialen Strömungsführung des Reaktionsmediums (3) aufweist.

## Beschreibung

Die Erfindung betrifft einen Reaktor zur Einstrahlung von ultraviolettem Licht in ein fluides Reaktionsmedium. Der Reaktor besteht wenigstens aus einem Gehäuse, welches einen rohrförmigen Hohlraum umschließt, mit einer Strahlungsquelle zur Erzeugung von ultraviolettem Licht und einem inneren Rohr, das mit dem Gehäuse einen insbesondere ringförmigen Bestrahlungsraum bildet, wobei der Bestrahlungsraum wenigstens mit einem Einlass und einem Auslass für das Reaktionsmedium verbunden ist und vom Reaktionsmedium in Längsrichtung des Rohres durchströmt wird, und wobei der Bestrahlungsraum Mittel zur Erzeugung einer zusätzlichen radialen Strömungsführung des Reaktionsmediums aufweist.

Die Sterilisation flüssiger Medien ist eine wesentliche Ausgangsvoraussetzung für den Einsatz bio- und lebensmitteltechnologischer Produktionsverfahren. Zielsetzung ist die zuverlässige und vollständige Abreicherung von Mikroorganismen und/oder Viren bei gleichzeitig weitestgehender Erhaltung der Wertstoffe. Sterilisiert werden sowohl die Einsatzstoffe (z.B. Nährmedien für Fermentationen) als auch Endprodukte (z.B. Milchprodukte oder pharmazeutische Wirkstoffproteine). In der Lebensmittelindustrie führen u.a. Aspekte der Haltbarkeitsverlängerung zur Anwendung der Sterilisationstechniken, während deren Einsatz in der pharmazeutischen Industrie durch strenge Qualitätssicherungsauflagen reglementiert ist. So werden zur Anwendung pharmazeutischer Produkte humanen oder tierischen Ursprungs mehrere, auf unterschiedlichen Wirkprinzipien basierende Virusinaktivierungsschritte gefordert, die eine Virusabreicherung um jeweils mindestens vier Zehnerpotenzen gewährleisten. Die Notwendigkeit zur Gewährleistung der "Virussicherheit" trifft selbstverständlich auch auf Pharmazeutika zu, die mit gentechnologischen Verfahren hergestellt werden.

Als produktschonendes Verfahren zur Virusabreicherung wird in der Literatur unter anderem die Bestrahlung mit ultraviolettem Licht vorgeschlagen. Die Behandlung von Plasma und Blutprodukten durch UV-Licht ist grundsätzlich bekannt. Bereits während des zweiten Weltkriegs wurden große Mengen Plasma gesammelt und mit UV-Licht bestrahlt. Die UV-Behandlung von Blutderivativen ist aber besonders hinsichtlich nicht umhüllter, hitzestabiler Viren interessant. Chin et al (Chin, S., Jin, R., Wang, X.L., Hamman, J., Gerard Marx, Xlaode Mou, Inger Andersson, Lars-Olof Lindquist, and Bernhard Horowitz (1997). Virucidal Treatment of Blood Protein Products with UVC Radiation. Photochemistry and Photobiology 65(3): 432-435.) konnten zeigen, dass eine Behandlung von Plasmaprodukten mit UV-Licht zur Inaktivierung von Hepatitis A- und Parvoviren führt.

Zielrichtung der UV-Bestrahlung ist die mutagene Veränderung des Erbmaterials der Mikroorganismen oder Viren, die oberhalb einer Mindestbestrahlungsdosis ihre Vermehrungsfähigkeit verlieren. Aufgabe der Erfindung ist es, hierfür eine sichere und optimal wirksame Vorrichtung zur Bestrahlung mit UV-Licht zu entwickeln.

Probleme bei der Verwendung von Reaktoren zur Einstrahlung von ultraviolettem Licht in flüssige Reaktionsmedien ergeben sich durch eine mit zunehmender Entfernung von der Strahlungsquelle exponentiell abnehmende Strahlungsintensität im zu behandelnden Medium. Mikroorganismen und Viren in einem größeren Abstand von der Strahlenquelle werden aus diesem Grund langsamer bzw. überhaupt nicht mehr abgetötet. Dieser Effekt, der mit zunehmendem Lichtabsorptionsvermögen des Mediums erheblich verstärkt wird, führt nach dem derzeitigen Stand der Technik zur Verwendung sehr großer Bestrahlungsoberflächen, wie man sie z.B. in Dünnschichtreaktoren vorfindet. Die im Einsatz befindlichen Dünnschichtreaktoren lassen sich nur schwer in den technischen Maßstab überführen, da die Konstanthaltung der Filmdicke bei der Maßstabsvergrößerung nur durch eine durchsatzproportionale Durchmesservergrößerung zu realisieren ist, was im technischen Maßstab zu nicht mehr handhabbar großen Reaktoren führt. Einen weiteren negativen Einfluss bildet das ungünstige Verweilzeitverhalten der nach Maßgabe der zumeist nur geringen Eindringtiefe der UV-Strahlung in das Reaktionsmedium notwendigerweise sehr dünnen und damit laminar strömenden Flüssigkeitsfilme, bei denen ein Austausch quer zur Hauptströmungsrichtung definitionsgemäß entfällt. Die wandnahen Schichten verweilen wegen des linear zur Wand bis auf Null abnehmenden Geschwindigkeitsprofiles wesentlich länger als die wandferneren Schichten. Um die zur Abtötung notwendige Mindestbestrahlungsdosis auch in der schneller fließenden wandfemen Flüssigkeitsschicht realisieren zu können, muss die mittlere Verweilzeit des Films angehoben werden. Dies aber führt zu einer erhöhten Strahlenbelastung und somit zur einer größeren Schädigung der Produkte.

Ebenfalls bekannt und beschrieben sind sogenannte Ringspaltreaktoren. Ein UV-Ringspaltreaktor üblicher Bauart besteht aus einem röhrenförmigen Metallgehäuse, in das ein, einen stabförmigen UV-Strahler enthaltendes Quarzrohr eingelassen ist, so dass ein ringspaltförmiger Raum gebildet wird. Bei diesem Reaktortyp fließt das Reaktionsmedium nur in axialer Richtung durch den Ringraum, was im Hinblick auf einen guten Stoffaustausch ähnlich wie bei den Dünnschichtreaktoren ebenfalls nicht vorteilhaft ist.

Die beschriebenen Nachteile der Reaktortypen sollten durch eine günstigere Strömungsführung überwunden werden können, die neben einem engen Verweilzeitspektrum auch einen guten Austausch in der Flüssigkeit senkrecht zur Hauptströmungsrichtung erlauben. Hierzu sind u.a. tangential angeströmte Ringspaltreaktoren vorgeschlagen worden Aus der EP 803 472 A1 ist z.B. ein Reaktor zur Einstrahlung von ultraviolettem Licht in ein Reaktionsmedium mit einem Ringraum als Bestrahlungszone, bei dem der Einlass so ausgebildet ist, dass das Reaktionsmedium tangential in den Ringraum eintritt.

Die Leistung eines Reaktors mit tangentialer Anströmung hat im Vergleich zu einem "klassischen" Ringspaltreaktor marginale Vorteile. Verfahrenstechnische Untersuchungen zeigen, dass das tangentiale Strömungsprofil infolge der Wandreibung bereits kurz nach dem Einlauf in ein axiales Profil umschlägt. Die zumindest für den Bereich der tangentialen Überströmung theoretisch postulierten Deanwirbel, mit denen der Queraustausch des Reaktionsmediums innerhalb des Ringspalts intensiviert werden soll, sind nach visuellen Studien und CFD-Untersuchungen (Strömungssimulation) nicht vorhanden, so dass tangential angeströmte Ringspaltreaktoren dieser Art zwar eine gewisse Verbesserung im Vermischungsverhalten aber dennoch keinen vollständigen Umsatz ermöglichen. Somit ist die Sekundärströmung und der damit verbundene verbesserte Stoffaustausch auf die einlaufnahen Zonen begrenzt.

Es konnte gezeigt werden, dass dieses Verhalten bei der Behandlung schwach absorbierender Reaktionsmedien (z.B. Wasserbehandlung) toleriert werden kann, da hierfür die Vermischung ausreichend ist und die UV-Dosis zur Umgehung dieses Nachteils erhöht werden kann. Für Anwendungen, die in Zusammenhang mit der Behandlung von Proteinlösungen stehen, schien dies nicht möglich zu sein, da die Proteine dabei irreversible Schäden erleiden würden.

Es ist folglich neu und überraschend, dass die Reaktoren der eingangs genannten Art auch für die Behandlung viruskontaminierter Proteinlösungen geeignet sind, wenn der Bestrahlungsraum über seine Länge Mittel zu einer zusätzlichen radialen Strömungsführung des Reaktionsmediums aufweist und insbesondere, wenn in Bezug auf den Durchmesser des Gehäuses eine bestimmte Reaktorlänge nicht überschritten wird. Das vorgeschlagene L/D - Verhältnis sollte bevorzugt kleiner 100 sein.

Wie aus der vorangegangen Diskussion deutlich wird, besteht die Aufgabe der Erfindung darin, Apparate der eingangs genannten Art mit einem optimierten und gleichmäßigeren Vermischungsverhalten für das Reaktionsmedium bereitzustellen.

Gegenstand der Erfindung ist ein Reaktor zur Einstrahlung von ultraviolettem Licht in ein fluides Reaktionsmedium mit einem Gehäuse, welches einen rohrförmigen Hohlraum umschließt, mit einer Strahlungsquelle zur Erzeugung von ultraviolettem Licht und einem inneren Rohr, das mit dem Gehäuse einen insbesondere ringförmigen Bestrahlungsraum bildet, wobei der Bestrahlungsraum wenigstens mit einem Einlass und einem Auslass für das Reaktionsmedium verbunden ist und vom Reaktionsmedium in Längsrichtung des Rohres durchströmt wird, dadurch gekennzeichnet, dass der Bestrahlungsraum Mittel zur Erzeugung einer zusätzlichen radialen Strömungsführung des Reaktionsmediums aufweist.

Die Vorrichtung (Reaktor) zur Einstrahlung von UV-Licht in Flüssigkeiten zeichnet sich aufgrund ihres optimalen und gleichmäßigen Vermischungsverhaltens durch einen besseren Stoffaustausch aus, wodurch eine sichere und effektive Sterilisation erreicht wird. Die Vorrichtung lässt sich gut in bestehende Anlagen integrieren und ist einfach zu reinigen. Ebenfalls von Vorteil ist die kompakte Bauweise der Vorrichtung.

Die Vorrichtung ist dadurch gekennzeichnet, dass in einem für UV-Licht durchlässigen Ringspaltkanal besondere Strömungsbedingungen erzeugt werden, die über die gesamte Kanallänge einen intensiven Stoffaustausch bewirken. Sie besteht z.B. aus einer UV-Strahlenquelle, die von einem Quarzschutzrohr (Strahlerhüllrohr) umgeben sein kann, und einem für UV-Licht durchlässigen Produktkanal (Bestrahlungsraum), durch den das Reaktionsmedium hindurchströmt. Das besondere Kennzeichen des Bestrahlungsraums ist eine intensive, über die gesamte Länge herrschende gleichförmige Quervermischung senkrecht zur Hauptrichtung der Produktströmung sowie eine durch turbulente Produktströmung eingeengte Verweilzeitverteilung.

Durch die Quervermischung wird gewährleistet, dass die von der Strahlenquelle entfernteren Flüssigkeitsschichten, die besonders bei stark lichtabsorbierenden Medien keine oder wenig UV-Strahlung erhalten, einen intensiven Austausch mit den UV-bestrahlten Schichten nahe der Strahlenquelle eingehen. Dies wird z.B. durch eine spezielle Strömungsführung in der Vorrichtung erreicht, durch die eine Vielzahl hintereinandergeschalteter, quasi-zellulärer Zirkulationsströmungen erzeugt wird. Hierdurch wird die notwendige Aufenthaltszeit der Produkte in den reaktiven Kanalschichten minimiert, was bei zuverlässiger Sterilisation bzw. Virusinaktivierung zur kleinst möglichen Schädigung der Produkte durch die Strahlenbelastung führt. Die Sekundärströmungen werden durch Rühren, beim Umströmen von Einbauten oder beim Durchströmen von spiralförmigen Kanälen erzeugt.

In einer bevorzugten Bauform ist der Reaktor so gestaltet, dass die Strahlungsquelle im inneren Rohr angeordnet ist und das innere Rohr für das ultraviolette Licht durchlässig ist.

Die Innenwand des Gehäuses weist dabei besonders bevorzugt eine Beschichtung mit einem UV-Strahlung reflektierenden Material auf.

In einer alternativen bevorzugten Bauform ist die Strahlungsquelle des Reaktors außerhalb des Gehäuses angeordnet und das Gehäuse für das ultraviolette Licht durchlässig.

Die Wand des inneren Rohres weist dann besonders bevorzugt eine Beschichtung mit einem UV-Strahlung reflektierenden Material auf.

Ein bevorzugtes Mittel zur Erzeugung einer zusätzlichen radialen Strömungsführung des Reaktionsmediums ist ein zylinderförmiger Rührer, insbesondere ein Zylinderrührer mit vorzugsweise 2 bis 10 Rührblättern, der im Bestrahlungsraum angeordnet ist.

Die zur schonenden UV-Behandlung vorzugsweise zu verwendenden kontinuierlich durchströmten Reaktoren mit Rührvorrichtung nutzen zur Quervermischung in vorteilhafter Weise die Ausbildung von Radial- und Taylorströmungen bzw. Kombinationen aus beiden. Taylorwirbel entstehen in Zylinderrührreaktoren durch Antreiben des inneren Zylinders. Bei Verwendung eines innen angetriebenen Zylinderrührers, der aus Gründen einer präzisen Rotationsbewegung vorteilhaft aus metallischen Werkstoffen anzufertigen ist, kann die UV-Bestrahlung auch durch den statischen äußeren Quarzglasmantel erfolgen, um den herum die UV-Röhren positioniert sind. Diese Vorrichtung erfordert zahlreiche UV-Strahler und verursacht damit einen größeren Bedienungsaufwand. Außerdem kann vom Gesamtangebot an UV-Strahlung nur derjenige Anteil nahezu verlustfrei genutzt werden, der rechtwinklig zur Glaswand aufgenommen wird, während die Strahlung in die anderen Einstrahlrichtungen durch Lichtbrechungen erheblich abgeschwächt wird. Bei einer zentrischen Bestrahlung durch einen den UV-Strahler eng umschließenden Innenzylinder entfallen diese Nachteile. Aus konstruktiven Gründen (zu nennen sind u. a. Probleme bei der steriltechnisch einwandfreien Abdichtung zwischen rotierendem Innenzylinder und Behälter sowie bei der berührungsfreien Lagerung des UV-Strahlers) ist jedoch der zur Erzeugung der Taylorwirbel benötigte Antrieb des inneren Glaszylinders nur mit technischem Aufwand zu realisieren.

Eine wesentlich einfachere Lösung zum Aufbau einer innen angetriebenen Tangentialströmung bietet ein mit kleinem Wandabstand von vorzugsweise 0,2 bis 20 mm um den Innenzylinder rotierender bevorzugter Zylinderrührer mit vorzugsweise 2 bis 10 Rührblättern. Die Rührblätter mit einer Breite von vorzugsweise 0,5 bis 30 mm lassen sich z.B. durch kavernenartige Einschnitte aus einem dünnwandigen Präzisionsrohr herausarbeiten.

CFD-Untersuchungen haben überraschenderweise gezeigt, dass durch den optionalen Einbau zusätzlicher Strömungsbrecher am äußeren Zylinderumfang, durch die die Tangentialströmung zugunsten von Radialströmungen mit dem Ziel einer weiteren Verbesserung des Queraustausches abgeschwächt wird, das Verweilzeitverhalten nicht nachteilig beeinflusst wird. Der Antrieb des Rührers erfolgt über einen externen Motor mit gleitringgedichteter Rührwelle oder vorzugsweise dichtungslos mittels einem elektromagnetisch gekoppelten Antrieb bzw. einem vom eintretenden Produktstrom angetriebenen Laufrad. Eine mechanisch intensivierte Reinigung der Reaktoren kann auf einfache Weise im geschlossenen Zustand bei eingeschaltetem Rührantrieb erfolgen oder nach Entfernen des inneren Glaszylinders durch Bürsten.

Aus der Patentschrift US 5 433 738 ist ein Bestrahlungsreaktor für die Bestrahlung von Wasser bekannt, der eine wendelförmige Leitung mit kreisrundem Querschnitt hat. Dieser weist aber keine hinreichende z.B. für die Virusinaktivierung notwendige Quervermischung auf, so dass seine Anwendung für die Virusinaktivierung zu unsicher ist.

Bei den nachfolgend beschriebenen, bevorzugten, kontinuierlich durchflossenen Ringspaltreaktoren mit statischen Einbauten kann auf bewegte Elemente vollständig verzichtet werden. Die Quervermischung kann dabei durch Deanwirbel, Freistrahlen und Produktumschichtung bewirkt werden. Deanwirbel treten in spiralförmigen Rohr- oder Kanalströmungen auf. Es hat sich nun gezeigt, dass die Verwendung von Spiralrohren mit zum Strahler hin abgeflachtem Querschnitt der Rohrflanken, z.B. bei Verwendung von Rechteck- oder D-Profilen, gegenüber den aus dem Stand der Technik bekannten runden Querschnitten zu bevorzugen sind, um eine Schwächung des eingetragenen UV-Lichts durch Lichtreflexionen zu vermeiden.

Bevorzugt ist daher eine Ausführung des Reaktors, bei der das Mittel zur Erzeugung einer zusätzlichen radialen Strömungsführung des Reaktionsmediums und der Bestrahlungsraum durch ein für UV-Strahlung durchlässiges im Querschnitt abgeflachtes Wendelrohr gebildet ist.

Besonders bevorzugt weist das Wendelrohr einen rechteckigen (vorzugsweise mit angerundeten Ecken), ovalen oder halbrunden Querschnitt auf.

Die Anwendung dieser Art von Reaktoren mit Wendelrohr bleibt aber im wesentlichen auf saubere Flüssigkeiten ohne extreme Qualitätsauflagen begrenzt, da die Wendelrohre einer mechanischen Reinigung nicht oder schwer zugänglich sind.

Bevorzugt lassen sich Spiralrohrströmungen auch durch Ausarbeiten von Spiralgängen aus einem von zwei berührungsnah ineinander schiebbaren Zylindern erzeugen.

Bevorzugter weiterer Gegenstand der Erfindung ist daher ein Reaktor, der dadurch gekennzeichnet ist, dass das Mittel zur Erzeugung einer zusätzlichen radialen Strömungsführung des Reaktionsmediums durch einen oder mehrere, insbesondere wendelförmige, Kanäle gebildet wird, die an der Innenwand und/oder der Außenwand des Bestrahlungsraums, bevorzugt an der Außenwand des Bestrahlungsraums umlaufend angeordnet sind.

Besonders bevorzugt haben die Kanäle ein rechteckförmiges (vorzugsweise mit abgerundeten Ecken), trapezförmiges oder halbrundes Querschnittsprofil.

Die Kanäle weisen insbesondere eine Tiefe von 1 bis 100 mm, bevorzugt von 2 bis 50 mm, und eine mittlere Breite von 1 bis 200 mm, bevorzugt von 2 bis 50 mm, im Querschnittsprofil auf.

Ganz besonders bevorzugt ist eine Bauform des Reaktors, in der die Kanäle wendelförmig ausgebildet sind und eine Steigung von 3 bis 30° (Steigungswinkel), bevorzugt von 8 bis 20°, aufweisen.

In der beschriebenen Variante des Reaktors mit Rührvorrichtung sowie auch mit den Kanälen wird der Bestrahlungsraum in Richtung der Rohrachse des inneren Rohres durchgehend offen ausgeführt.

Da die Bestrahlung vorzugsweise durch den inneren Zylinder erfolgt, wird die wendelförmige Vertiefung mit einer Schnitttiefe von vorzugsweise 1 bis 40 mm aus dem äußeren Zylinder ausgearbeitet.

Spiralrohrströmungen lassen sich auch mittels speziell geformter Schläuche erzeugen, die erfindungsgemäß über einen Zylinder gezogen werden. Bevorzugt ist folglich auch ein Reaktor, bei dem die Außenwand des Bestrahlungsraums und die Kanäle durch einen Wellschlauch gebildet werden.

Eine vollständige Abdichtung der Innenflanke der Spirale gegen die Glaswand ist möglich, aber nicht unbedingt erwünscht, um ein Fouling in den schlecht durchströmten Zwickeln um die Berührungszonen zwischen Schlauch (Außenzylinder) und Quarzglas zu vermeiden. Durch Gewährleistung eines Mindestabstandes zwischen Flanke und Glaswand, unter Bildung eines kleinen Ringspaltes, wobei der Abstand günstigerweise im Bereich zwischen 0,1 und 0,8 mm liegt, wird eine Schlupfströmung induziert, die als Freistrahl in den darüber liegenden Gewindegang eintritt, wo sie mit den Deanwirbeln überlagert einen zusätzlichen Betrag zur Intensivierung der Quervermischung leistet. Ein günstiger Abstand zwischen Schlauch (Außenzylinder) und Glasrohr beträgt bei einer Breite des Wendelkanals von 2 bis 20 mm, insbesondere von 0,1 bis 1 mm.

Bei nur axial durchströmten Reaktoren wird auf die durch die Spiralrohrströmung induzierten Deanwirbel vollständig verzichtet. Stattdessen werden durch zusätzliche Einbauten besonders bevorzugt ca. 10 bis 100 mm hohe Ringspaltkammern mit einer lichten Weite von vorzugsweise 3 bis 15 mm und einer Höhe von 2 bis 6 mal der Breite gebildet, deren obere und untere Begrenzungen bezogen auf die Kammertiefen einen Spaltabstand von vorzugsweise 5 bis 40 % zum inneren Glaszylinder besitzen. Der Produktstrom wird beim Passieren der Begrenzungen in einer Spaltströmung auf eine höhere Geschwindigkeit beschleunigt. Dieser als Freistrahl in die nächste Kammer eingeleitete Produktstrom, saugt am Eintritt umgebende Flüssigkeit an und beschleunigt diese unter Reduzierung der eigenen Geschwindigkeit. Hierdurch wird ein kräftiger Sekundärwirbel induziert. Tritt der Freistrahl am inneren Glaszylinder aus, kommt es im äußeren Bereich des Ringspaltes zu einer Rückströmung. Wird die Scheibe gegen den Glaszylinder gedichtet und ein Zulaufspalt am Außenradius des Ringspaltes geöffnet, liegt die Rückströmzone in der Nähe des Innenzylinders. Bei einer abwechselnden Anordnung der Zulaufspalte am Innen- und Außenradius des Ringspaltes kann somit neben dem erzeugten Wirbel eine mehrfache alternierende Umschichtung des Produktes von innen nach außen und umgekehrt erreicht werden.

Der Reaktor kann so ausgeführt sein, dass der Einlass so ausgebildet ist, dass das Reaktionsmedium tangential, radial oder axial in den Bestrahlungsraum eintritt. Die Ausführung mit tangentialem Einlass wird besonders bevorzugt eingesetzt, insbesondere bei der Bauform mit offen durchgehender Bestrahlungszone.

In einer bevorzugten Variante des Reaktors ist wenigstens ein UV-Sensor mit Messeinrichtung zur Messung der UV-Intensität der Strahlungsquelle am Reaktor angebracht, insbesondere im oberen oder unteren Bereich des Reaktors, z.B. in der Nähe des Einlasses und/oder des Auslasses.

Eine weitere bevorzugte Variante des Reaktors weist wenigstens einen UV-Sensor mit Messeinrichtung zur Messung der UV-Intensität im Bestrahlungsraum, insbesondere im unteren oder oberen Bereich des Reaktors, auf beispielsweise in der Nähe des Einlasses und/oder des Auslasses des Reaktors.

Die Nutzung des Reaktors richtet sich auf eine Vielzahl unterschiedlicher Anwendungen zur UV-Bestrahlung und/oder Sterilisierung von Flüssigkeiten.

Weiterer Gegenstand der Erfindung ist Verwendung des erfindungsgemäßen Reaktors zur Bestrahlung und Sterilisierung fluider Medien und insbesondere Mikroorganismen und/oder Viren enthaltender Flüssigkeiten, bevorzugt von Nahrungsmitteln, besonders bevorzugt Milch- oder Fruchtsaftprodukten oder Trinkwasser, chemischen oder pharmazeutischen Produkten, insbesondere bevorzugt von Virus-Vakzinen, gentechnisch erzeugten Wirkstoffen oder Proteinen, z.B. Wirkstoffen oder Proteinen aus transgenen Tieren oder Pflanzen und von Blutplasma oder aus Blutplasma gewonnenen Produkten.

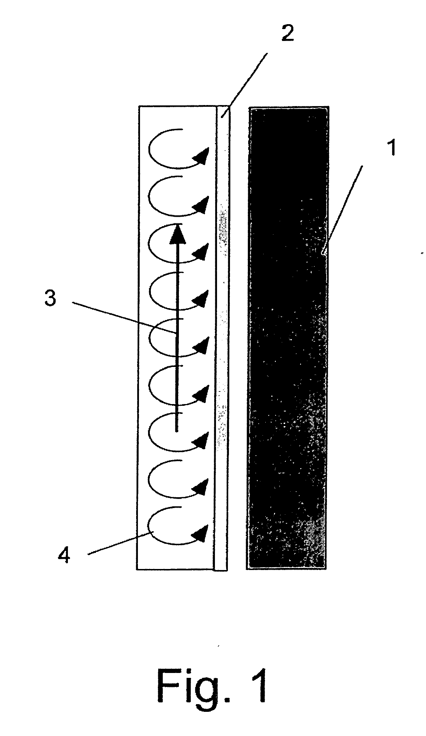

Im folgenden werden Ausführungsbeispiele der Erfindung anhand von Figuren näher erläutert. Es zeigen
- Figur 1: einen schematischen Schnitt durch einen Teil eines Bestrahlungsraums
- Figur 2: einen Ringspaltrührreaktor mit Ankerrührer, zentrischer UV-Bestrahlung und Magnetantrieb im Längsschnitt
- Figur 2a: ein vergrößertes Detail aus Figur 2
- Figur 3: einen kavernenartig ausgearbeiteten Ankerrührer aus Figur 2 mit Magnet
- Figur 4a: einen Laufradantrieb für den Ankerrührer nach Figur 2 im Längsschnitt B-B
- Figur 4b: den Laufradantrieb nach Figur4a im Querschnitt A-A
- Figur 5: einen Wendelrohrreaktor mit halbrundem Rohrquerschnitt
- Figur 5a: ein vergrößertes Detail aus Figur 5
- Figur 6: einen Wendelrohrreaktor mit rechteckigem Rohrquerschnitt
- Figur 6a: ein vergrößertes Detail aus Figur 6
- Figur 7: einen demontierbaren Spiralrohrreaktor mit Kanälen im Längsschnitt
- Figur 7a: ein vergrößertes Detail aus Figur 6
- Figur 8: einen am Innendurchmesser des Ringspaltes angetriebenen Freistrahlreaktor mit trapezförmigen Kanälen im Längsschnitt
- Figur 8a: ein vergrößertes Detail aus Figur 8
- Figur 9: einen Umschichtreaktor mit alternierender Produktzugabe am Innen- und Außendurchmesser des Ringspaltes
- Figur 9a: ein vergrößertes Detail aus Figur 9
- Figur 10: den oberen Teil eines Ringspaltreaktors mit UV-Sensoren

### Beispiele

### Beispiel 1

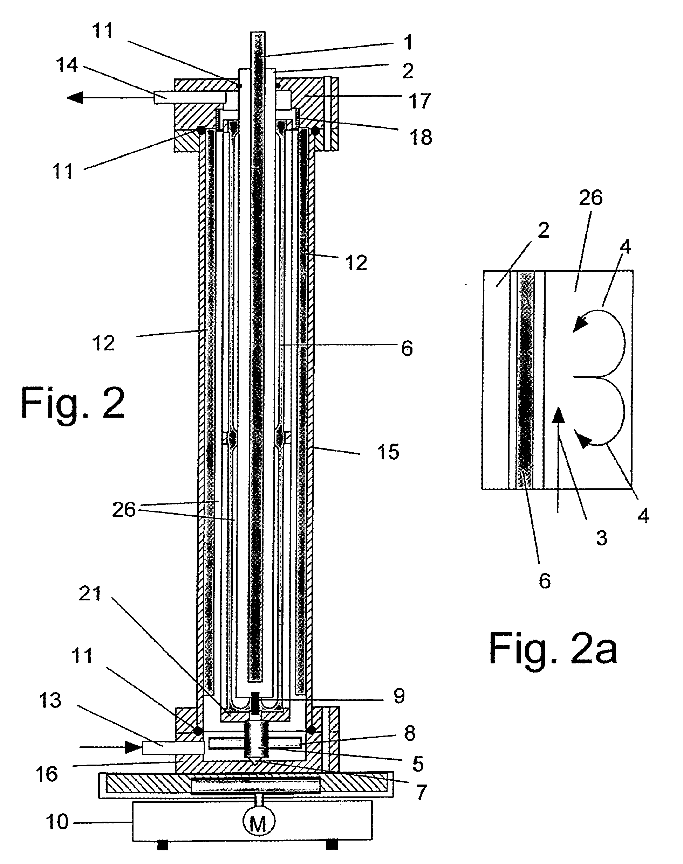

Der UV- Reaktor gemäß Figur 2 und Figur 2a besteht aus einer zentrisch eingebauten UV-Lampe 1 (Hg-Leuchtstoffröhre) mit dem Aussendurchmesser 25 mm und der Länge von 850 mm, die durch einen nach oben geöffneten Quarzglasmantel 2 (inneres Quarzglasrohr) mit Innendurchmesser 26 mm ohne Produktberührung eingeführt und entnommen werden kann. Die offene Seite des Rohres 2 ist durch einen O-Ring 11 im Kopfdeckel 17 steriltechnisch einwandfrei gedichtet. Um das Glasrohr 2 rotiert in geringem Abstand von 0,5 mm ein mit 8 Rührblättern bewehrter Ankerrührer 6. Dieser wird im Kopfdeckel 17 durch ein Gleitlager 18 und im Bodendeckel 16 durch eine Zentrierspitze 7 gelagert. Der Antrieb erfolgt durch Magnetkupplung, indem die von einem externen Magnetrührwerk 10 zur Verfügung gestellte Leistung auf einen quer zur Rührwelle 5 montierten Gegenmagneten 8 berührungs- und somit dichtungslos übertragen wird. Zur Gewährleistung des Wandabstandes zwischen Ankerrührer 6 und Glasrohr 2 wird die achsensymmetrische Lage des Rohres 2 mittels eines Zentrierstiftes 9 gesichert, der innerhalb der Welle 5 geführt wird. Der Ringspalt 26 (Bestrahlungsraum) von 5 mm, in dem die Inaktivierungsreaktion stattfindet, wird nach innen begrenzt durch die Außenwand des Rohres 2 und nach außen durch die optional mit vier 3 mm breiten Strombrechern 12 bewehrte Innenwand des Mantelrohres 15, an dessen beiden Enden Flansche zur Befestigung des Boden- 16 und den Kopfdeckels 17 angeschweißt sind.

Das Produkt wird mit einem Durchsatz von 150 - 300 1/h in den Stutzen 13 des Bodendeckels eingespeist und am Kopfdeckel 17 über Stutzen 14 abgezogen.

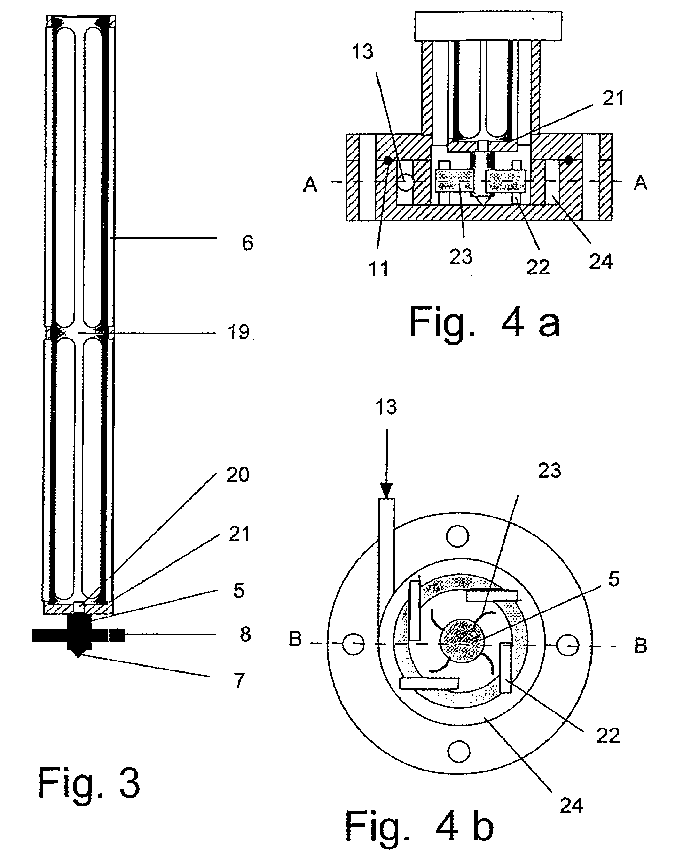

Der Zylinderrührer 6 mit 8 Blättern in der Form gemäß Figur 3 wird aus einem Präzisionsrohr mit dem Außendurchmesser 31 mm und der Wandstärke 0,8 mm durch Ausschneiden 16 kavernenartiger Aussparungen angefertigt. Die Aussparungen erstrecken sich aus Stabilitätsgründen nicht durchgängig über die gesamte Rührerlänge, sondern reichen bis zu den Stegverbindungen 19. Der Ankerrührer 6 ist über eine Scheibe 21 mit der Rührwelle 5 verbunden. Die bis in die Welle 5 hineinreichende Zentrieröffnung 20 dient zur Zentrierung des unten geschlossenen Rohres 2. Der Rührer seinerseits wird im Bodendeckel 16 über eine Zentrierspitze 7 zentriert.

### Beispiel 2

Der als Alternative zur Magnetkupplung in der Bauform nach Figur 2 eingesetzte ebenfalls dichtungslose Schaufelradantrieb für den Ankerrührer 6 gemäß der Figur 4a (Schnitt B-B) und 4b (Schnitt A-A) besteht aus 4 auf der Rührwelle 5 befestigten, konvex gebogenen Schaufelrädern 23 mit einer Höhe von 10 mm. Der Durchmesser des Schaufelrades beträgt 39 mm. Der Antrieb erfolgt durch das in einen vorgelagerten Ringraum 24 tangential eingeleitete Produkt, das über vier gegen den Innenraum tangential angestellte Spalte 22 mit einer Spaltweite von jeweils 0,8 mm auf die Antriebsschaufeln geleitet wird.

### Beispiel 3

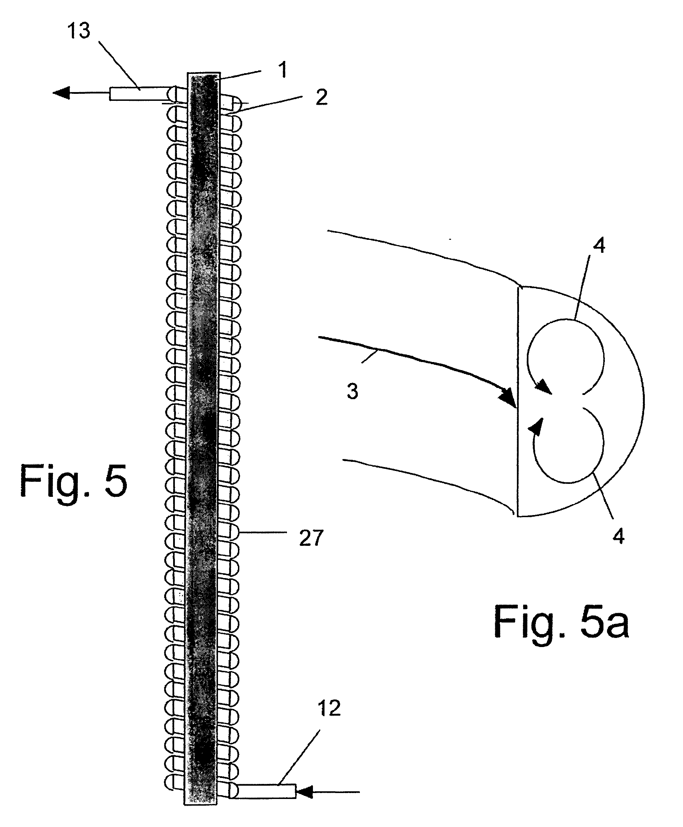

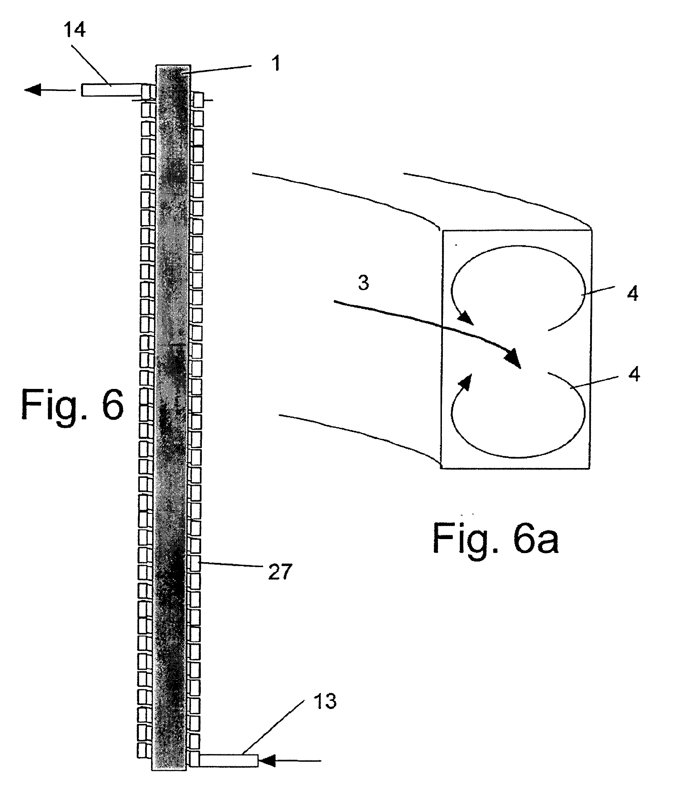

Der UV- Reaktor gemäß Figur 5 und Figur 5a besteht aus einer UV-Lampe 1 mit dem Durchmesser 25 mm, um die ein Quarzglasrohr 27 mit halbrundem Querschnitt von 8 mm Durchmesser und 4 mm Radius in geringem Abstand gewickelt ist. Der UV- Reaktor gemäß Figur 6 und Figur 6a besteht aus einer UV-Lampe 1 mit dem Durchmesser 25 mm, um die ein Quarzglasrohr 27 mit rechteckigem Querschnitt von 8 mm Breite und 4 mm Tiefe in geringem Abstand gewickelt ist. Rechtwinklig zur Spiralströmung 3 werden Sekundärwirbel 4, sog. Dean-Wirbel induziert, die eine Umwälzung des zu bestrahlenden Gutes im Rohr 27 zur Folge haben.

### Beispiel 4

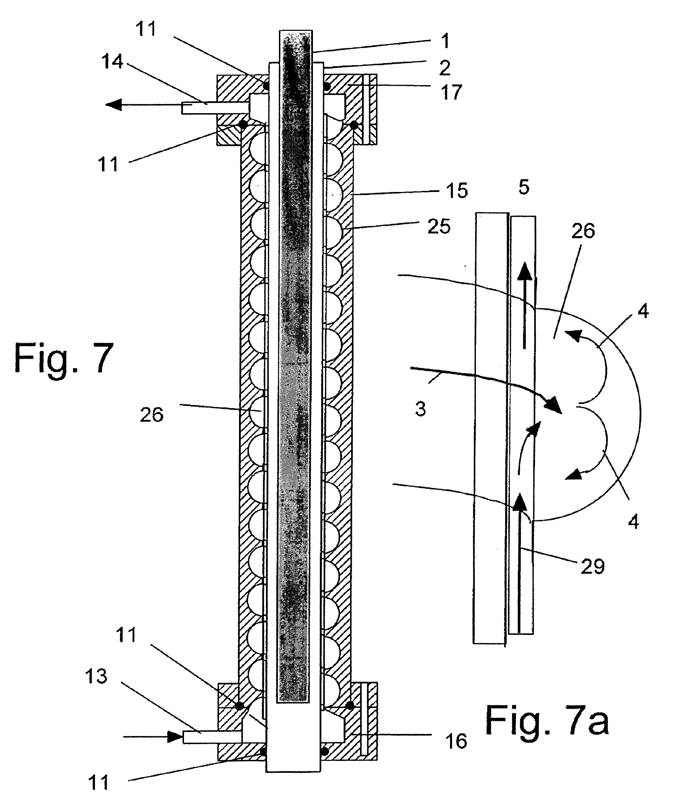

Der UV-Reaktor gemäß Figur 7 und Figur 7a besteht aus einem UV-Strahler 1 mit umgebendem, durchgängigem, am Kopf- 17 und Bodendeckel 16 mit O-Ringen 11 gegen den Reaktionsraum 26 gedichtetem Quarzglasrohr 2, das auf der Außenseite spiralförmig vom Produkt überströmt wird. Die Kontur der spiralförmigen Strömung wird durch den Kanal 25 im Außenzylinder 15 vorgegeben. Zur Erzeugung des Kanals 25 ist in den Außenzylinder 15 mit einer Tiefe von 4 mm und einer Breite von 6 mm ein Rundgewindegang eingeschnitten sind. Der kleinste Abstand zwischen Glasrohr 2 und Zylinder beträgt 0,5 mm. Dieser Spalt erlaubt zur Verringerung der Fouling-Problematik einem Teil der Produktströmung als Freistrahl 29 in den darüber liegenden Strömungskanal einzutreten. Die nahezu senkrecht auf die spiralförmige Hauptströmung 3 gerichtete Freistrahlströmung 29 (siehe Figur 7a) führt zu einer weiteren Verstärkung des ersten der beiden durch die Spiralströmung erzeugten Sekundärwirbel 4. Mit dieser Anordnung wird eine gegenüber dem Wendelrohr deutlich verbesserte gleichmäßige Bestrahlung des Produktes erreicht.

### Beispiel 5

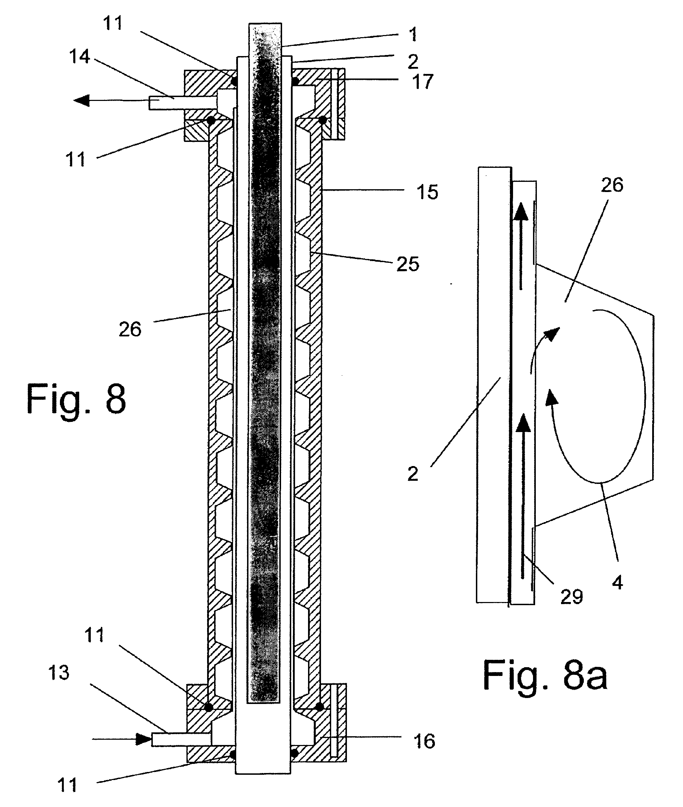

Der UV-Reaktor gemäß Figur 8 und Figur 8a besteht aus einem UV-Strahler 1 mit umgebendem, durchgängigem, am Kopf- 17 und Bodendeckel 16 mit O-Ringen 11 gegen den Reaktionsraum 26 gedichtetem Quarzglasrohr 2, das auf der Produktseite in axialer Richtung überströmt wird. Durch die sägezahnförmige Kontur der Kanäle 25' des Außenzylinders 15 werden eine Vielzahl voneinander abgegrenzter Ringspalträume mit einer Höhe von 30 mm und einer Spaltweite von 4 mm für die Ausbildung der Sekundärwirbel 4 geschaffen (vergl. Figur 8a). Die Sekundärwirbel werden durch Freistrahlen 29 angetrieben, die im 0,7 mm breiten Ringspalt beim Eintritt des Produkstromes in die Kanäle 25' erzeugt werden.

### Beispiel 6

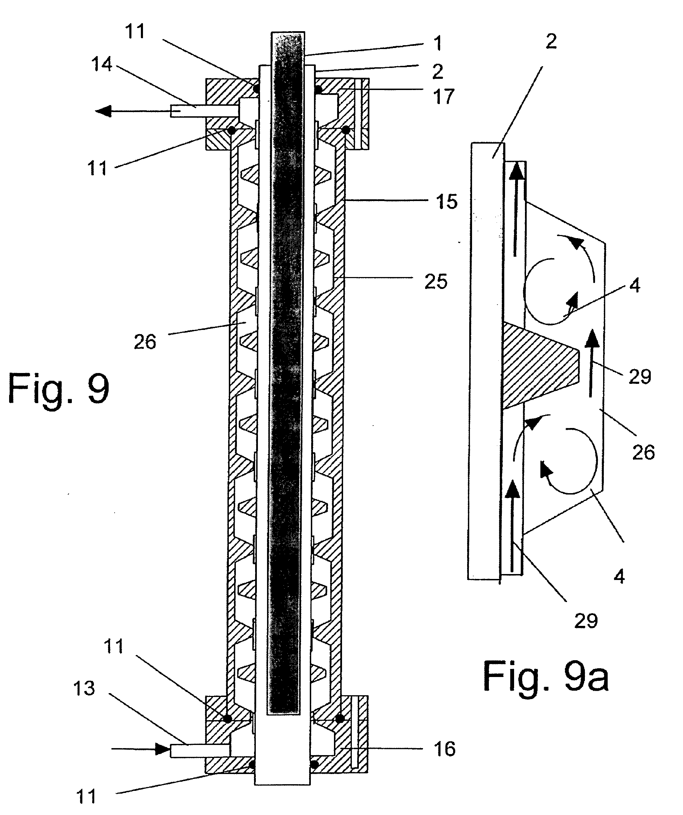

Der UV-Reaktor gemäß Figur 9 und Figur 9a besteht aus einem UV-Strahler 1 mit umgebendem, durchgängigem, am Kopf- 17 und Bodendeckel 16 mit O-Ringen 11 gegen den Reaktionsraum 26 gedichtetem Quarzglasrohr 2, das auf der Produktseite in axialer Richtung überströmt wird. Durch die spezielle Kontur der Kanäle 25' des Außenzylinders werden Kammern mit einer Breite von 4 mm und einer Höhe von 30 mm gebildet, in denen mit Freistrahlen 61 angetriebene gegenläufige Sekundärwirbel 4 erzeugt werden, die zu einer alternierenden Umschichtung des Produktes von der Innenseite zur Außenseite der Kammern führen. Die axialen Freistrahlen werden in 0,7 mm breiten Ringspalten auf dem inneren und äußeren Umfang der Kammern erzeugt.

### Beispiel 7

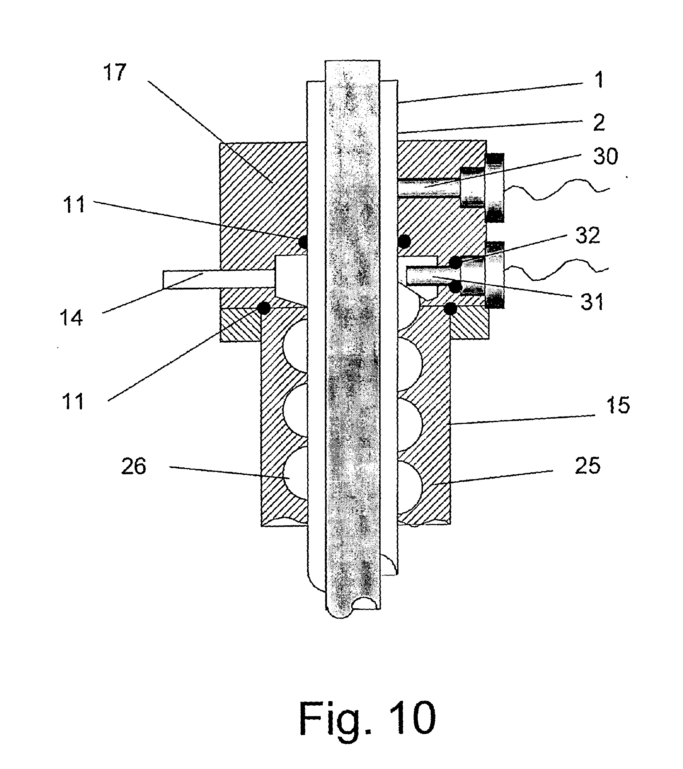

Der UV-Reaktor gemäß Figur 10 (Teilansicht) ist gegenüber dem Reaktor nach Figur 7 dahingehend modifiziert, dass der Spalt zwischen Glasrohr 2 und Außenzylinder 15 weggelassen ist.

Zusätzlich ist im Kopfbereich 17 ein UV-Sensor 30 angebracht, der direkt die von dem UV-Strahler 1 abgegebene UV-Strahlung misst. Hiermit wird z.B. eine Regelung der UV-Intensität ermöglicht.

Ein zweiter UV-Sensor 31 ist im Bestrahlungsraum angeordnet, um "Fouling-Prozesse" im Reaktor beobachten zu können.

Am Fuß des Reaktors befinden sich weitere zwei UV-Sensoren für die prinzipiell gleichen oben angegebenen Zwecke (in Figur 10 nicht gezeichnet).

## Patentansprüche

1. Reaktor zur Einstrahlung von ultraviolettem Licht in ein fluides Reaktionsmedium (3) mit einem Gehäuse (15), welches einen rohrförmigen Hohlraum umschließt, mit einer Strahlungsquelle (1) zur Erzeugung von ultraviolettem Licht und einem inneren Rohr (2), das mit dem Gehäuse (15) einen insbesondere ringförmigen Bestrahlungsraum (26) bildet, wobei der Bestrahlungsraum (26) wenigstens mit einem Einlass (13) und einem Auslass (14) für das Reaktionsmedium (3) verbunden ist und vom Reaktionsmedium (3) in Längsrichtung des Rohres (2) durchströmt wird, **dadurch gekennzeichnet, dass** der Bestrahlungsraum (26) Mittel zur Erzeugung einer zusätzlichen radialen Strömungsführung des Reaktionsmediums (3) aufweist, wobei das Mittel in einem oder mehreren wendelförmigen Kanälen (25) besteht, die an der Innenwand und/oder der Außenwand der Bestrahlungsraums (26) umlaufend angeordnet sind, wobei die Kanäle (25) ein mit abgerundeten Ecken versehenes, rechteckförmiges, trapezförmiges oder helbrundes Querschnittsprofil aufweisen.

2. Reaktor nach Anspruch 1, **dadurch gekennzeichnet, dass** die Strahlungsquelle (1) im inneren Rohr (2) angeordnet ist und das innere Rohr (2) für das ultraviolette Licht durchlässig ist.

3. Reaktor nach Anspruch 2, **dadurch gekennzeichnet, dass** die Innenwand des Gehäuses (15) eine Beschichtung mit einem UV-Strahlung reflektierenden Material aufweist.

4. Reaktor nach Anspruch 1, **dadurch gekennzeichnet, dass** die Strahlungsquelle (1) außerhalb des Gehäuses (15) angeordnet ist und das Gehäuse (15) für das ultraviolette Licht durchlässig ist.

5. Reaktor nach Anspruch 4, **dadurch gekennzeichnet, dass** die Wand des inneren Rohres (2) eine Beschichtung mit einem IJV-Strahlung reflektierenden Material aufweist.

6. Reaktor nach Anspruch 1 bis 5, **dadurch gekennzeichnet, dass** die Kanäle (25) eine Tiefe von 1 bis 100 mm und eine mittlere Breite von 1 bis 200 mm im Querschnittsprofil aufweisen.

7. Reaktor nach einem der Ansprüche 1 bis 6, **dadurch gekennzeichnet, dass** die Kanäle (25) wendelförmig ausgebildet sind und eine Steigung von 3 bis 30° aufweisen.

8. Reaktor nach einem der Ansprüche 1 bis 7, **dadurch gekennzeichnet, dass** der Bestrahlungsraum (26) in Richtung der Rohrachse des inneren Rohres (2) durchgehend offen ausgerührt ist.

9. Reaktor nach einem der Ansprüche 1 bis 8, **dadurch gekennzeichnet, dass** die Außenwand des Bestrahlungsraums (26) und die Kanäle (25) durch einen Wellschlauch gebildet werden.

10. Reaktor nach einem der Ansprüche 1 bis 9, **dadurch gekennzeichnet, dass** der Einlass (13) so ausgebildet ist, dass das Reaktionsmedium tangential in den Bestrahlungsraum (26) eintritt.

11. Reaktor nach Anspruch 10, **dadurch gekennzeichnet, dass** das Verhältnis Länge des Reaktors zum Durchmesser des Gehäuses auf L/D < 100 begrenzt ist.

12. Reaktor nach einem der Ansprüche 1 bis 8, **dadurch gekennzeichnet, dass** der Einlass (13) so ausgebildet ist, dass das Reaktionsmedium radial in den Bestrahlungsraum (26) eintritt.

13. Reaktor nach einem der Ansprüche 1 bis 8, **dadurch gekennzeichnet, dass** der Einlass (13) so ausgebildet ist, dass das Reaktionsmedium axial in den Bestrahlungsraum (26) eintritt.

14. Reaktor nach einem der Ansprüche 1 bis 13, **dadurch gekennzeichnet, dass** wenigstens ein UV-Sensor (30) mit Messeinrichtung zur Messung der UV-Intensität der Strahlungsquelle (1) am Reaktor angebracht ist, insbesondere im oberen oder unteren Bereich des Reaktors.

15. Reaktor nach einem der Ansprüche 1 bis 14, **dadurch gekennzeichnet, dass** wenigstens ein UV-Sensor (31) mit Messeinrichtung zur Messung der UV-Intensität im Bestrahlungsraum (26), insbesondere im unteren oder oberen Bereich des Reaktors, angebracht ist.

16. Verwendung eines Reaktors nach einem der Ansprüche 1 bis 15 zur Bestrahlung und Sterilisierung fluider Medien und insbesondere Mikroorganismen und/oder Viren enthaltender Flüssigkeiten, besonders bevorzugt von Nahrungsmitteln, bevorzugt Milch- oder Fruchtsaftprodukten oder Trinkwasser, chemischen oder pharmazeutischen Produkten, insbesondere bevorzugt von Virus-Vakzinen, gentechnisch erzeugten Wirkstoffen oder Proteinen, Wirkstoffen oder Proteinen aus transgenen Tieren oder Pflanzen und von Blutplasma oder aus Blutplasma gewonnenen Produkten.
